# EUROPÄISCHE PATENTSCHRIFT

(11) **EP 1 191 379 B1**
(45) Veröffentlichungstag und Bekanntmachung des Hinweises auf die Patenterteilung: **19.03.2008**
(21) Anmeldenummer: 01120468.2
(22) Anmeldetag: 28.08.2001
(51) Int. Cl.: G02B 21/00, G02B 7/00

(54) **Mikrosokop mit einer Handhabe bzw. Handgriff für ein Mikroskop**
Microscope with a handle particularly handle for a microscope
Microscope avec poignée , particulierement poignée pour un microscope

(30) Priorität: 22.09.2000 CH 18972000
(43) Veröffentlichungstag der Anmeldung: 27.03.2002
(73) Patentinhaber: Leica Microsystems Schweiz AG, 9435 Heerbrugg (CH)
(72) Erfinder: Strobel, Peter, Kathu, Phuket, 83150 (TH); Sollberger, Fritz, 8494 Bauma (CH)
(74) Vertreter: Rosenich, Paul

(56) Entgegenhaltungen:
- DE-A- 19 640 993
- FR-A- 2 541 573
- JP-A- 2000 139 949
- US-A- 5 121 655
- US-A- 5 694 815
- US-A- 5 861 983
- US-A1- 2002 014 562
- US-B1- 6 434 416
- PATENT ABSTRACTS OF JAPAN Bd. 2000, Nr. 22, 9. März 2001 (2001-03-09) & JP 2001 120573 A (OLYMPUS OPTICAL CO LTD), 8. Mai 2001 (2001-05-08)

## Beschreibung

Die Erfindung betrifft die Handgriffe an einem Mikroskop - einerseits von Ihrer Art der Befestigung am Mikroskopgehäuse und andererseits von Ihrer Anzahl und vom Ort der Befestigung.

Insbesondere betrifft sie einen Handgriff für ein Mikroskop, der in ergonomischer und komfortabler Weise an einem Mikroskop angebracht werden kann, welches an einem geeigneten beweglichen Stativ aufgehängt ist, so dass das Mikroskop auch unter sterilen Bedingungen im Raum bewegt werden kann. Zu solchen Bewegungen gehören die Richtungen Auf/Ab, Vor/Rück, Seitwärts, Drehen, Schwenken, Neigen usw.

Herkömmliche Handgriffe sind in verschiedensten Formen und Montagearten bekannt geworden. Meistens sind sie paarweise (links und rechts) am Mikroskopgehäuse angeschraubt oder an Stangen ausgebildet, die am Schwenkträger eines Mikroskops befestigt sind und dieses somit nur indirekt bewegen.

Die Offenlegungsschrift DE-A1-196 40 993 zeigt z.B. solch ein diametral angeordnetes, gebogenes Handgriffs-Paar.

Bei der Steuerung eines Mikroskops legt der Anwender seine beiden Hände an die Griffstücke herkömmlicher Handgriffe, betätigt gegebenenfalls elektrische Schalter o.dgl., um Bremsen o.dgl. zu lösen und bewegt dann mit Muskelkraft das Mikroskop in die gewünschte Stellung. Geübte Anwender können besonders leichtgängig gelagerte Mikroskope, wie beispielsweise Operationsmikroskope an einem besonderen Stativ (OHS) der Anmelderin, die meisten Bewegungen des Mikroskops auch mit einer Hand durchführen, da die elektrischen Schaltelemente an beiden Handgriffen - links wie rechts - angeordnet sind.

Die paarweisen Handgriffe sind häufig - vergleichbar einer Motorradlenkstange - horizontal erstreckt, so dass ein Anwender das Mikroskop zwischen seinen ausgebreiteten Händen anwenden kann.

Nachteilig ergibt sich daraus allerdings eine Benutzung eines an sich freien Raumes durch die Handgriffe. Dies führt vor allem bei Aufbauten mit Assistentenanschlüssen für Assistenten zu Platzproblemen. Vor allem die an Stangen befestigten Handgriffe erhöhen nachteiligerweise das Gesamtgewicht des Mikroskopaufbaus, der durch das Stativ getragen werden muss.

Der Erfindung liegt somit als eine Aufgabe zugrunde, eine verbesserte Griffanordnung bzw. Ausgestaltung zu finden, die die erwähnten Probleme vermeidet.

Gelöst wird diese Aufgabe durch die Merkmale des Anspruchs 1. Der Ersatz von zwei Handgriffen durch einen Handgriff, der an einer zentralen Stelle des Mikroskopkörpers angebracht oder lösbar anbringbar ist und sich in wenigstens zwei Raumrichtungen des Koordinatensystems erstreckt, führt einerseits zu einer deutlichen Gewichtsreduktion und andererseits zu der Möglichkeit, das Mikroskop mit nur einer Hand zu bedienen. Die erwähnten Platzprobleme für die Assistenten sind somit beseitigt. Die Erstreckung in wenigstens zwei Raumrichtungen gibt dem Anwender die Möglichkeit, mit nur einer Hand verschiedene Schwenkmomente aufzubringen und dabei günstige Hebelmomente anzuwenden.

Das Patent US 5,694,815 offenbart gegen die Kraft einer gefederten Kugel lösbare Handgriffe, die jedoch auch paarweise an den Seiten eines Mikroskopträgers angeordnet sind und nicht weiter gesichert sind.

Die erfindungsgemäße Einhandbedienung durch einen einzigen Handgriff erleichtert einem Anwender die Lageveränderung des Mikroskops bei gleichzeitiger Benutzung der anderen Hand für andere Tätigkeiten.

Herkömmliche Handgriffe sind mehr oder weniger ergonomisch geformt, um dem Bedienpersonal einen guten Griff zu erlauben.

Jedoch auch bei der Anwendung von nur einem Griff kann es, wie schon bei herkömmlichen paarweisen Griffen, problematisch sein, dass sie im Betrieb - nach ihrer Benutzung zur Verstellung des Mikroskops u.U. im Weg sind. Dies tritt z.B. auf, wenn mehrere Personen um das Mikroskop arbeiten, oder wenn Positionen eingenommen werden, bei denen die Bedienperson den Platz, an dem sich der Griff befindet, benötigen.

Als eine zweite Aufgabe, die der Erfindung zugrunde liegt, ist somit die Verbesserung dieses Problems. Gelöst wird diese Aufgabe durch die Schaffung eines vom Mikroskopkörper lösbaren Handgriffes. Da dabei die Ergonomie und die Geschwindigkeit beim Lösen groß gehalten werden soll, ist erfindungsgemäß bevorzugt eine Einhandbedienung vorgesehen.

Die Erfindung ist jedoch nicht auf lösbare Einhandgriffe eingeschränkt. Sie umfasst vielmehr auch Varianten mit paarweisen Handgriffen, die einzeln oder gemeinsam erfindungsgemäß lösbar sind.

Erfindungsgemäß ist bei den lösbaren Handgriffen am Griffstück ein Montageteil ausgebildet, das mit einem Haltestück oder mit einer gegengleichen Ausbildung des Mikroskopgehäuses kooperiert.

Selbstverständlich umfasst die Erfindung auch alle vertauschten Ausbildungen (Männchen mit Weibchen getauscht).

Entscheidend ist die einfache Einhandlösbarkeit des Verbindungsmechanismus. Es kommen Montageglieder als Klemmhebel, Knebelhebel, Montageschrauben oder Montagemuttern mit steilen Gewinden oder andere Elemente zur Anwendung, die nur wenige oder nur Bruchteile von Umdrehungen benötigen um zu verriegeln oder zu entriegeln, wie z.B: Bajonettschließteile (Männchen bzw. Weibchen) o.dgl.

Erfindungsgemäß sind dabei sowohl Lösungsvarianten umfasst, die zwei Finger zur Bedienung erfordern oder insbesondere auch solche, die mit einem Finger auskommen. Dabei ist die Bedienung mittels Daumen bevorzugt, weil dieser Finger in der Regel die größte Kraft aufbringen kann.

Von Vorteil sind spezielle Aufbauten, bei denen Klemmhebel zum Einsatz gelangen, die in der einen Stellung die Fixierung und in der anderen Stellung die Loslösung definieren. Solche Klemmhebel sind besonders schnell zu bedienen und erlauben - wenn mit dem Daumen bedient - eine hohe Schließ- bzw. Lösekraft.

Theoretisch genügt eine Einpunktbefestigung, sofern das Montageteil satt am Mikroskopgehäuse anliegt. Bevorzugt ist jedoch bei einer Ausführung erfindungsgemäß eine Mehrpunktbefestigung vorgesehen, bei der neben einer Verriegelungsstelle eine Raststelle vorgesehen ist. Die Raststelle kann z.B. über einen Rastzapfen oder über eine Welle verfügen, der bzw. die in gegengleiche Ausnehmungen eingreift, so dass nach dem Verbinden der Raststelle ein Schwenken des Griffteils in die Verriegelungsstelle möglich ist.

Die Erfindung ermöglicht erstmals auch das Montieren von Handgriffen über einem Drape. D.h., dass ein Mikroskop, das in der Regel nicht optimal sterilisierbar ist, durch das Drape voll geschützt wird und ein optimal sterilisierbarer Handgriff darüber angebracht wird. Um dies zu ermöglichen, ist bei einer besonderen Ausführung der Erfindung zwischen dem Montageteil des Handgriffs und dem Haltestück am Mikroskopgehäuse ein ausreichender Hub (Spiel) vorgesehen.

Um Toleranzen auszugleichen sind bei einer besonderen Ausbildung elastische oder federbelastete Elemente vorgesehen.

Die Ausbildung des erfindungsgemäßen Griffs unterliegt den bekannten Aufgabenstellungen und Lösungen, wobei die erfindungsgemäße Möglichkeit neu hinzukommt, den Griff als Einhandbediengriff zu benutzen. D.h., dass nicht nur die Loslösung und die Montage des Griffs, sondern auch die Führung des Mikroskops durch nur eine Hand erfolgen kann. Demzufolge ist es von Vorteil, wenn der Griff nach unten gekrümmt ist, da damit der ergonomischen Handhaltung einerseits, andererseits aber auch der Möglichkeit gedient ist, Schwenkkräfte aufzubringen.

Bevorzugt ist der Handgriff aus einer Reihe von unterschiedlich geformten Griffen wählbar, um dem jeweiligen Bedienpersonal an Komfort maximal entgegenzukommen. Dies war bisher mit fest montierten Griffen gar nicht erst möglich.

Gemäß einer anderen Weiterbildung umfasst der Handgriff auch elektrische Bedienelemente, die über Steckkontakte im Bereich des Montageteils mit den Schaltkreisen am Mikroskopgehäuse verbindbar sind. Diese Steckkontakte sind vorzugsweise so integriert, dass die elektrische Verbindung gleichzeitig mit der mechanischen Verbindung erfolgt.

Besonders kommt die erfindungsgemäße Stärke des neuen Konzepts zum Tragen, wenn am Mikroskopgehäuse mehr als nur eine Stelle zur Griffmontage zur Verfügung steht, weil dann je nach Bedarf der richtige Montageort gewählt werden kann. Und dies insbesondere auch unter Operationsbedingungen, wo rasche Reaktionen und kurze Umstellzeiten wichtig sind.

Es kommen reine Klemmverbindungen zur Anwendung, bei denen durch Klemm- bzw. Haftreibung genügende Verbindungskräfte aufgebracht werden können. Das Lösen erfolgt bei solchen Aufbauten z.B. durch eine ruckartige Bewegung.

Eine typische Variante wäre z.B., eine Haftverbindung mittels Magnetkraft, die in Form von Dauermagneten oder von Elektromagneten aufgebracht werden.

Durch die Erfindung und ihre Weiterbildungsvarianten können somit verbessert die Bedürfnisse z.B. speziell in der Mikrochirurgie, in der Hals-Nasen-Ohrenchirurgie und in allen anderen auch industriellen Anwendungen, von Mikroskopen berücksichtigt werden.

Anhand von Zeichnungen soll die Erfindung beispielhaft näher erläutert werden.

Es zeigen dabei
- Fig. 1: ein Mikroskop, wie es beispielsweise an einem Stativ schwenkbar gehalten ist mit einem neuartigen Handgriff;
- Fig.2: einen Schnitt entlang der Linie A-A der Fig. 3 des neuen Handgriffs;
- Fig. 3: einen neuen Handgriff in der Sicht von oben;
- Fig. 4: ein Haltestück, das an einem Mikroskopgehäuse befestigt werden kann;
- Fig. 5: eine Schrägansicht auf einen erfindungsgemäßen Handgriff;
- Fig.6: den vergleichbaren Handgriff nach Fig. 5, jedoch von der Mikroskopseite her gesehen;
- Fig. 7: den Handgriff von Fig. 6 in schräger Seitenansicht in Vormontagestellung;
- Fig. 8: den Handgriff von Fig. 6 in schräger Rückansicht;
- Fig. 9: den Handgriff von Fig.6 in schräger Rückansicht mit geschlossener Spindel und
- Fig. 10: den Handgriff von Fig. 6 in schräger Frontansicht.

Gleiche Bauteile tragen in der nachfolgenden Figurenbeschreibung gleiche Bezugszeichen, unterschiedliche Bauteile tragen unterschiedliche Bezugszeichen.

Unterschiedliche Bauteile mit gleichen Funktionen tragen gleiche Bezugszeichen mit unterschiedlichen Indizes.

Die Figuren werden übergreifend beschrieben.

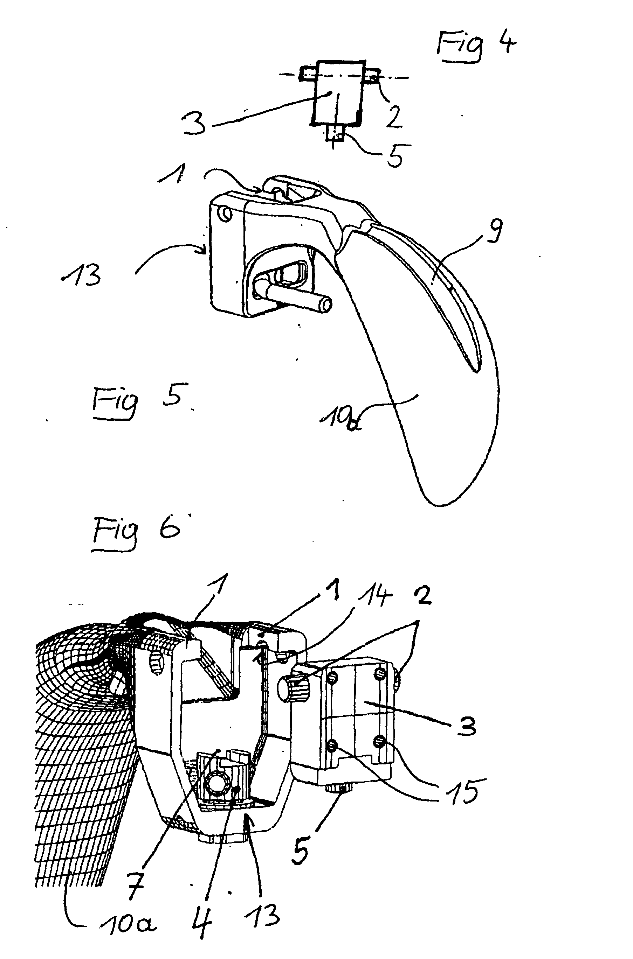

Ein Mikroskopkörper 12 mit einem Hauptobjektiv 8 verfügt an einer zentralen Stelle über ein Haltestück 3, das am Mikroskopkörper 12 ausgebildet oder mit diesem beispielsweise durch Schrauben in Befestigungsbohrungen 15 (Fig. 6) fest verbunden ist.

Das Haltestück 3 dient zur Aufnahme eines Montageteils 11 mit dem Griffteil 10 des neuartigen Handgriffs. Solche Haltestücke könnten mehrere am Mikroskopkörper oder am Schwenkträger oder am Stativ oder an anderen Orten montiert sein. Das Haltestück 3 verfügt über zwei Lagerzapfen bzw. eine Welle 2 am oberen Ende des Haltestücks 3 und einen Druckbolzen 5 am unteren Ende des Haltestücks 3.

Sowohl Welle 2 als auch Druckbolzen 5, als u.U. auch die äußere Form des Haltestücks dienen zur Aufnahme einer Montagevorrichtung 13 eines Montageteils 11 des Handgriffs. Der Montageteil ist einstückig mit einem Griffteil 10 verbunden, der einer Bedienperson das Lageverändern des Mikroskops erlaubt

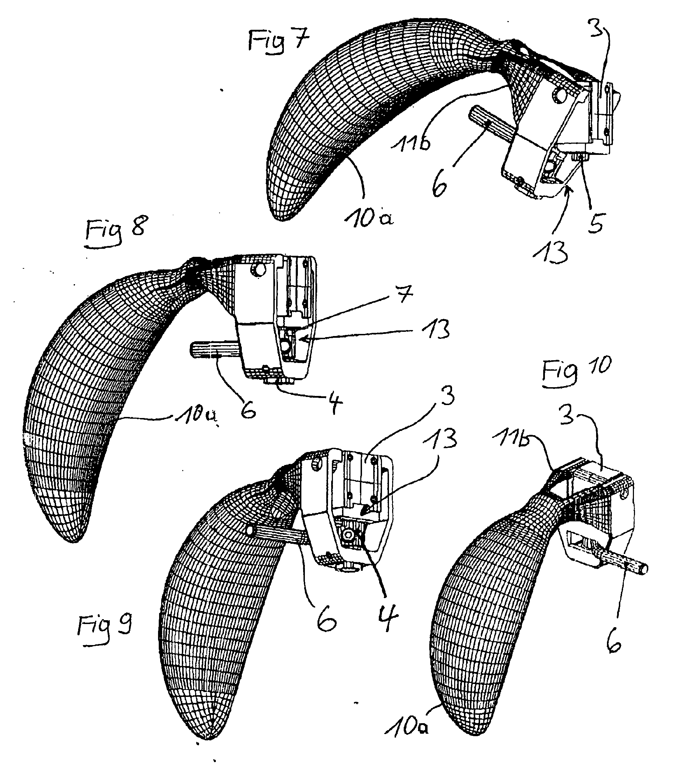

Die Montagevorrichtung 13 umfasst eine schraubstockartige Spindel 4, die einen Schlitz 7 zur Verriegelung aufweist, der mit dem Druckbolzen 5 kooperiert und diesen festspannt, sofern die Spindel um ihre Achse gedreht wird. Der Schlitz 7 verfügt dazu über Seitenwände, die in Montagestellung den Druckbolzen 5 gegen die Löserichtung verriegeln. Gleichzeitig ist die Spindel 4 jedoch in einem Gewinde gelagert, das so ausgebildet ist, dass es bei einem Drehen der Spindel 4 in Schließrichtung zu einer Druckbeaufschlagung des Druckbolzens 5 In seiner Axialrichtung kommt, wodurch sich das Montageteil 11 an dem Haltestück 3 festkrallt. Dazu sind gegenüber der Spindel 4 in der Montagevorrichtung zwei fluchtende Halblager 1 vorgesehen, die in der Montagestellung mit der Welle 2 kooperieren. Dazu haben sie schalenförmige Ausnehmungen 14, die um die Welle 2 schwenken können, wie im Vergleich der Fig. 7 und Fig. 8 dargestellt.

Fig.8 zeigt die Spindel 4 in dem Zustand, in dem der Schlitz 7 bereit ist, den Druckbolzen 5 aufzunehmen, während Fig. 9 jene Spindelstellung zeigt, in der der Druckbolzen 5 verriegelt ist.

Die Spindel 4 ist durch einen Klemmhebel 6 bedienbar, der aus der Montagevorrichtung 13 in Richtung Griffstück 10 ragt. Der Klemmhebel kann in dem Ausführungsbeispiel problemlos mit einem Finger, z.B. mit dem Daumen bedient werden, um eine Ver- oder Entriegelung der Spindel 4 mit dem Druckbolzen 5 zu ermöglichen.

### Verriegelungsvorgang:

Das Halblager 1 der Montagevorrichtung 13 wird in leichter Schräglage von oben auf die Welle 2 des Haltestücks 3 aufgesetzt und dann leicht nach unten auf Anschlag geschwenkt, so dass die Spindel 4 der Montagevorrichtung 13 unter den Druckbolzen 5 des Haltestücks 3 zu liegen kommt. Wird in dieser Position der Klemmhebel 6 und mit ihm die Spindel 4 um ca. 90° verdreht, verriegeln die Seitenwände des Schlitzes 7 der Spindel 4 den Druckbolzen 5, und die Bodenfläche des Schlitzes 7 drückt von unten auf den Druckbolzen 5. Somit wird einerseits das Halblager 1 auf die Welle 2 gedrückt und andererseits ein Formschluss mit dem Druckbolzen 5 erzeugt, wodurch der Haltegriff fest auf dem Haltestück 3 fixiert wird.

Als Spindel sind auch andere nicht dargestellte Elemente denkbar, z.B. Exzenter, Keile etc. Zur Kompensation eines Hubes oder Spieles bzw. von Toleranzen - z.B., wenn der Haltegriff über einem Drape montiert werden soll - kann die Spindel 4 bzw. der Druckbolzen 5 abgefedert sein.

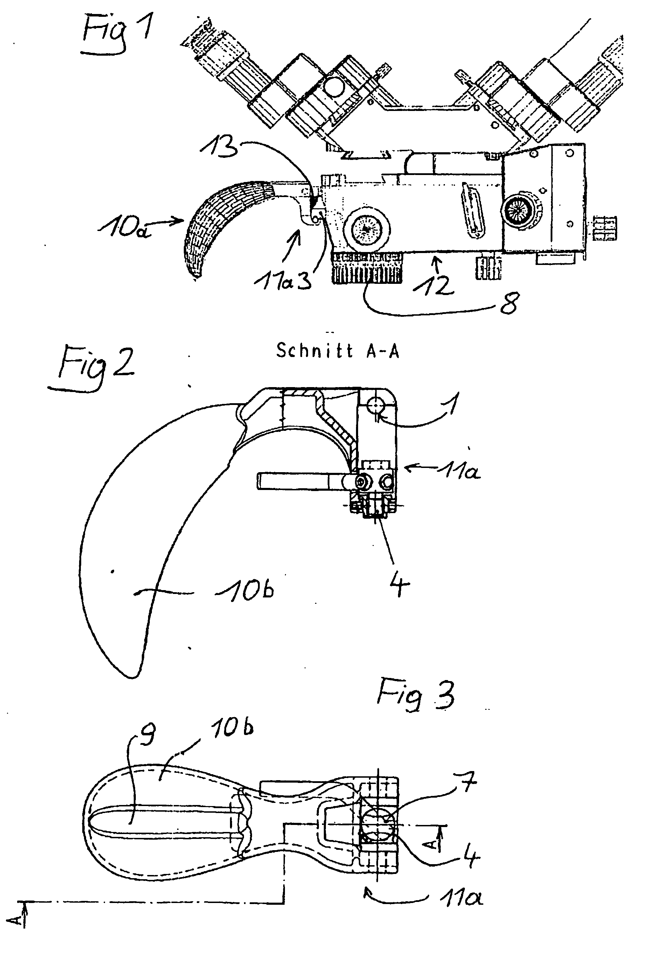

Die Griffstücke 10b gemäß der Fig. 2-Fig.5 sind unterschiedlich von den übrigen Griffstücken 10a, die mit einer deutlichen Bananenform mit strukturierter Oberfläche ausgebildet sind. Die Leichtbauweise des Griffstücks 10b sieht zur Gewichtserleichterung eine Freistellung 9 vor und ist hohl, wie Fig. 2 entnommen werden kann.

### Bezugszeichenliste

- 1: Halblager (Teil eines Lagers zum Einsetzen eines Verriegelungsteils)
- 2: Welle
- 3: Haltestück
- 4: Spindel, schraubstockartig
- 5: Druckbolzen
- 6: Klemmhebel
- 7: Schlitz
- 8: Hauptobjektiv
- 9: Freistellung
- 10: Griffstück
- 11: Montageteil
- 12: Mikroskopgehäuse
- 13: Montagevorrichtung
- 14: Ausnehmung, schalenförmig

## Patentansprüche

1. Mikroskop mit wenigstens einem Haltestück (3) an einem Mikroskopkörper (12) oder wenigstens einer Befestigungs-Ausformung am Mikroskopkörper (12) mit einem Handgriff zum Lageverändern des Mikroskops an einem Schwenkträger, wobei der Handgriff einen Griffteil (10) und ein Montageteil (11) zur Montage des Griffteils (10) am Mikroskopkörper (12) und einer Montagevorrichtung (13) am Montageteil (11) umfasst, wobei die Montagevorrichtung (13) ein Montageglied (6) umfasst, das durch eine Hand am Griffteil (10) einhändig offen- oder schließbar ist, sodass die Montagevorrichtung (13) und damit der Handgriff mit dem Mikroskopkörper (12) direkt - oder indirekt über das Haltestück (3) - verbindbar bzw. von ihm lösbar ist, **dadurch gekennzeichnet, dass** das Montageglied als Klemmhebel (6) oder als Knebelhebel oder als Montageschraube - bzw. -mutter oder als Bajonettschließteil ausgebildet ist und dass das Montageglied aus dem Montageteil (11) so hervor ragt, dass es mit einem oder zwei Fingern einer Hand am Griffteil (10) bedienbar ist wobei das Montageglied bzw. der Klemmhebel (6) mit einer schraubstockartigen Spindel (4) verbunden ist, die an ihrer freien Stirnseite einen Schlitz (7) aufweist, der mit einem zapfenförmigen Druckbolzen (5) des Haltestücks (3) oder mit der entsprechenden Befestigungs-Ausformung am Mikroskopgehäuse (12) kooperiert, sodass ein Verschwenken des Montageglieds bzw. Klemmhebels (6) zu einem formschlüssigen Festspannen bzw. Loslösen des Druckbolzens (5) am bzw. vom Montageteil (11) führt.

2. Mikroskop nach Anspruch 1, **dadurch gekennzeichnet, dass** ein Haltestück (3) oder eine Befestigungs-Ausformung am Mikroskopkörper (12) an einer zentralen Stelle des Mikroskopkörpers (12) angeordnet ist.

3. Mikroskop nach Anspruch 2, **dadurch gekennzeichnet, dass** das Haltestück (3) oder die Befestigungs-Ausformung am Mikroskopkörper (12) an der zentralen Stelle des Mikroskopkörpers (12) der einzige Montageort für den Handgriff ist.

4. Mikroskop nach einem der vorhergehenden Ansprüche, **dadurch gekennzeichnet, dass** das Montageglied (6) mit einem Fingervorzugsweise mittels Daumen - bedienbar ist

5. Mikroskop nach einem der vorhergehenden Ansprüche, **dadurch gekennzeichnet, dass** das Haltestück (3) bzw. die Befestigungs-Ausformung am Mikroskopgehäuse (12) eine Welle (2) oder wenigstens einen Lagerbolzen umfasst, der mit einer gegengleichen Ausbildung am Montageteil (11) so kooperiert, dass es beim Schließen der Montagevorrichtung (13) zu einer wenigstens Zweipunkt-Verrastung des Montageteils (11) am Halteteil (3) bzw. an der Befestigungs-Ausformung am Mikroskopgehäuse (12) kommt.

6. Mikroskop nach einem der vorhergehenden Ansprüche, **dadurch gekennzeichnet, dass** die Montagevorrichtung (13) zur Kompensation von Toleranzen wenigstens einen Exzenter, Kniehebel, Keil o.dgl. aufweist.

7. Mikroskop nach einem der vorhergehenden Ansprüche, **dadurch gekennzeichnet, dass** die Montagevorrichtung (13)bzw. das Montageteil (11) wenigstens ein federbelastetes oder ein elastisches Element aufweist oder elastisch ausgebildet ist

8. Mikroskop nach einem der vorhergehenden Ansprüche, **dadurch gekennzeichnet, dass** der Hub (Spiel) zwischen Montageteil (11) und Haltestück (3) bzw. Befestigungs-Ausformung des Mikroskopgehäuses (12) so gewählt ist, dass der Handgriff über einem Drape montierbar ist, sodass das Drape zwischen Montageteil (11) und Haltestück (3) bzw. Mikroskopgehäuse (12) einklemmbar ist.

9. Mikroskop nach einem der vorhergehenden Ansprüche, **dadurch gekennzeichnet, dass** die Montagevorrichtung (13) am Mikroskopgehäuse (12) und das Haltestück (3) am Handgriff angeordnet sind.

10. Mikroskop nach einem der vorhergehenden Ansprüche, **dadurch gekennzeichnet, dass** der Griffteil (10) bananenförmig ausgebildet und vorzugsweise - in Montagelage - nach unten gekrümmt ist.

11. Mikroskop nach einem der vorhergehenden Ansprüche, **dadurch gekennzeichnet, dass** wenigstens der Griffteil (10) antihaftend, z.B. durch Lotosblüteneffkt, und/oder sterilisierbar beschichtet bzw. ausgebildet ist.

12. Mikroskop nach einem der vorhergehenden Ansprüche, **dadurch gekennzeichnet, dass** am Griffteil (10) elektrische Schaltelemente zur Mikroskopsteuerung angeordnet sind **und dass** das Montageteil (11) elektrische Steckkontakte zur Verbindung mit den Schaltkreisen am Mikroskopkörper (12) aufweist.

13. Mikroskop nach einem der vorhergehenden Ansprüche, **dadurch gekennzeichnet, dass** der Handgriff aus einer Serie von unterschiedlich geformten Handgriffen wählbar ist, bei der die Montageteile (11) gleich, die Griffteile (10) jedoch unterschiedlich geformt sind - insbesondere zur Berücksichtigung von unterschiedlichen Handgrößen und/oder zur Berücksichtigung einer linken oder einer rechten Griffhand.

14. Mikroskop nach einem der vorhergehenden Ansprüche, **dadurch gekennzeichnet, dass** der Handgriff in Leichtbauweise, als Hohlkörper - vorzugsweise aus Leichtmetall oder Kunststoff - aufgebaut ist.

15. Mikroskop nach einem der vorhergehenden Ansprüche, **dadurch gekennzeichnet, dass** der Handgriff über Rastelemente (4, 7, 13) verfügt, die mit gegengleichen Gegenrastelementen (2,5) am Mikroskopgehäuse (12) ver- bzw. entrastbar ist.

16. Mikroskop nach Anspruch 15, **dadurch gekennzeichnet, dass** die Rastelemente (2, 4, 5, 7, 13) so ausgebildet sind, dass sie im Montagezustand über Klemmreibung und/oder über kraft- oder formschlüssige Wirkkräfte verfügen.

## Claims

1. Microscope with at least one holding piece (3) on a microscope body (12) or at least one fastening projection on the microscope body (12) with a hand-grip for varying the position of the microscope on a pivoted support, the hand-grip comprising a grip part (10) and a mounting part (11) for mounting the grip part (10) on the microscope body (12) and a mounting device (13) on the mounting part (11), the mounting device (13) comprising a mounting element (6) which can be opened or closed with one hand by a hand on the grip part (10), such that the mounting device (13) and thus the hand-grip can be connected to or released from the microscope body (12) directly - or indirectly via the holding piece (3), **characterized in that** the mounting element is constructed as a clamping lever (6) or as a tommy lever or as a mounting screw or mounting nut, or as a bayonet closing part, and **in that** the mounting element projects from the mounting part (11) such that it can be operated with one or two fingers of one hand on the grip part (10), the mounting element or the clamping lever (6) being connected to a vice-like spindle (4) which has on its free end face a slot (7) which cooperates with a pin-shaped thrust bolt (5) of the holding piece (3), or with the corresponding fastening projection on the microscope housing (12) such that pivoting of the mounting element or clamping lever (6) leads to self-closed clamping or releasing of the thrust bolt (5) on or from the mounting part (11).

2. Microscope according to Claim 1, **characterized in that** a holding piece (3) or a fastening projection is arranged on the microscope body (12) at a central point of the microscope body (12).

3. Microscope according to Claim 2, **characterized in that** the holding piece (3) or the fastening projection on the microscope body (12) at the central point of the microscope body (12) is the sole mounting location for the hand-grip.

4. Microscope according to one of the preceding claims, **characterized in that** the mounting element (6) can be operated with one finger - preferably by means of the thumb.

5. Microscope according to one of the preceding claims, **characterized in that** the holding piece (3) or the fastening projection on the microscope housing (12) comprises a shaft (2) or at least one bearing bolt which cooperates with a matching formation on the mounting part (11) such that closure of the mounting device (13) produces an at least two-point latching of the mounting part (11) on the holding part (3) or on the fastening projection on the microscope housing (12).

6. Microscope according to one of the preceding claims, **characterized in that** to compensate tolerances the mounting device (13) has at least one eccentric cam, toggle lever, wedge or the like.

7. Microscope according to one of the preceding claims, **characterized in that** the mounting device (13) or the mounting part (11) has at least one spring-loaded or one elastic element, or is of elastic construction.

8. Microscope according to one of the preceding claims, **characterized in that** the travel (play) between the mounting part (11) and holding piece (3) or fastening projection of the microscope housing (12) is selected such that the hand-grip can be mounted over a drape such that the drape can be clamped between the mounting part (11) and holding piece (3) or the microscope housing (12).

9. Microscope according to one of the preceding claims, **characterized in that** the mounting device (13) is arranged on the microscope housing (12), and the holding piece (3) is arranged on the hand-grip.

10. Microscope according to one of the preceding claims, **characterized in that** the grip part (10) is of banana-shaped construction and preferably curved downwards in the mounted position.

11. Microscope according to one of the preceding claims, **characterized in that** at least the grip part (10) is coated or constructed antiadhesively, for example by means of the lotus blossom effect, and/or in a sterilizable fashion.

12. Microscope according to one of the preceding claims, **characterized in that** electric switching elements for microscope control are arranged on the grip part (10), and **in that** the mounting part (11) has electric plug-in contacts for connecting to the circuits on the microscope body (12).

13. Microscope according to one of the preceding claims, **characterized in that** the hand-grip can be selected from a series of differently shaped hand-grips of which the mounting parts (11) are identically shaped, but the grip parts (10) are differently shaped - in particular in order to take account of different hand sizes and/or to take account of a left or a right gripping hand.

14. Microscope according to one of the preceding claims, **characterized in that** the hand-grip is constructed in a lightweight fashion as a hollow body, preferably made from light metal or plastic.

15. Microscope according to one of the preceding claims, **characterized in that** the hand-grip has latching elements (4, 7, 13) which can be latched to or unlatched from matching mating latching elements (2, 5) on the microscope housing (12).

16. Microscope according to Claim 15, **characterized in that** the latching elements (2, 4, 5, 7, 13) are constructed such that in the mounted state they have clamping friction and/or force-closed or self-closed active forces.

## Revendications

1. Microscope comprenant au moins une pièce de fixation (3) sur un corps de microscope (12) ou au moins une formation de fixation sur le corps de microscope (12), avec une poignée pour faire varier la position du microscope sur un support pivotant, la poignée présentant une partie de poignée (10) et une partie de montage (11) pour le montage de la partie de poignée (10) sur le corps de microscope (12) et d'un dispositif de montage (13) sur la partie de montage (11), le dispositif de montage (13) comprenant un organe de montage (6) qui peut être ouvert ou fermé d'une main saisissant la partie de poignée (10), de sorte que le dispositif de montage (13) et de ce fait la poignée puisse être connecté(e) au corps de microscope (12) directement ou indirectement par le biais de la pièce de fixation (3), ou puisse en être détaché(e), **caractérisé en ce que** l'organe de montage est réalisé sous forme de levier de serrage (6) ou sous forme de levier à manette ou sous forme de vis ou d'écrou de montage ou sous forme de partie de fermeture à baïonnette, et **en ce que** l'organe de montage dépasse de la partie de montage (11) de telle sorte qu'il puisse être commandé avec un ou deux doigts d'une main saisissant la partie de poignée (10), l'organe de montage ou le levier de serrage (6) étant connecté à une broche (4) en forme d'étau, qui présente une fente (7) sur son côté frontal libre, laquelle coopère avec un boulon de pression (5) en forme de tourillon de la pièce de fixation (3) ou avec la formation de fixation correspondante sur le corps du microscope (12), de sorte qu'un pivotement de l'organe de montage ou du levier de serrage (6) conduise à un serrage fixe par coopération de forme ou un relâchement du boulon de pression (5) sur ou de la partie de montage (11).

2. Microscope selon la revendication 1, **caractérisé en ce que** l'on dispose une pièce de fixation (3) ou une formation de fixation sur le corps de microscope (12) en un point central du corps de microscope (12).

3. Microscope selon la revendication 2, **caractérisé en ce que** la pièce de fixation (3) ou la formation de fixation sur le corps de microscope (12) au point central du corps de microscope (12) est le seul lieu de montage pour la poignée.

4. Microscope selon l'une quelconque des revendications précédentes, **caractérise en ce que** l'organe de montage (6) peut être commandé d'un doigt, de préférence du pouce.

5. Microscope selon l'une quelconque des revendications précédentes, **caractérisé en ce que** la pièce de fixation (3) ou la formation de fixation sur le corps de microscope (12) comprend un arbre (2) ou au moins un boulon de palier, qui coopère avec une réalisation diamétralement opposée sur la partie de montage (11), de telle sorte que lors de la fermeture du dispositif (13), il se produise un encliquetage en au moins deux points de la partie de montage (11) sur la pièce de fixation (3) ou sur la formation de fixation sur le corps de microscope (12).

6. Microscope selon l'une quelconque des revendications précédentes, **caractérisé en ce que** le dispositif de montage (13) présente, pour la compensation des tolérances, au moins un excentrique, un levier à genouillère, une clavette ou similaire.

7. Microscope selon l'une quelconque des revendications précédentes, **caractérisé en ce que** le dispositif de montage (13) ou la partie de montage (11) présente au moins un élément sollicité par ressort ou un élément élastique, ou est réalisé(e) sous forme élastique.

8. Microscope selon l'une quelconque des revendications précédentes, **caractérisé en ce que** la course (jeu) entre la partie de montage (11) et la pièce de fixation (3) ou la formation de fixation du corps de microscope (12) est choisie de telle sorte que la poignée puisse être montée par-dessus un champ opératoire, de sorte que le champ opératoire puisse être serré entre la partie de montage (11) et la pièce de fixation (3) ou le corps de microscope (12).

9. Microscope selon l'une quelconque des revendications précédentes, **caractérisé en ce que** le dispositif de montage (13) est disposé sur le corps de microscope (12) et la pièce de fixation (3) est disposée sur la poignée.

10. Microscope selon l'une quelconque des revendications précédentes, **caractérisé en ce que** la partie de poignée (10) est réalisée en forme de banane et est de préférence courbée vers l'intérieur dans la position de montage.

11. Microscope selon l'une quelconque des revendications précédentes, **caractérisé en ce qu'**au moins la partie de poignée (10) présente un revêtement antiadhérant, par exemple à effet lotus, et/ou peut être réalisée ou revêtue de manière stérilisable.

12. Microscope selon l'une quelconque des revendications précédentes, **caractérisé en ce que** l'on dispose sur la partie de poignée (10) des éléments de commutation pour la commande du microscope, et **en ce que** la partie de montage (11) présente des contacts électriques enfichables pour la connexion aux circuits de montage sur le corps du microscope (12).

13. Microscope selon l'une quelconque des revendications précédentes, **caractérisé en ce que** la poignée peut être choisie parmi une série de poignées de formes différentes, dans lesquelles les parties de montage (11) sont identiques, mais les parties de poignée (10) peuvent être formées différemment, en particulier pour tenir compte des différentes tailles de mains et/ou pour tenir compte d'une prise à main gauche ou à main droite.

14. Microscope selon l'une quelconque des revendications précédentes, **caractérisé en ce que** la poignée est construite suivant une construction légère, sous forme de corps creux, de préférence en métal léger ou en plastique.

15. Microscope selon l'une quelconque des revendications précédentes, **caractérisé en ce que** la poignée dispose d'éléments d'encliquetage (4, 7, 13), qui peuvent être encliquetés ou désencliquetés avec des éléments d'encliquetage conjugués (2, 5) sur le corps de microscope (12).

16. Microscope selon la revendication 15, **caractérisé en ce que** les éléments d'encliquetage (2, 4, 5, 7, 13) sont réalisés de telle sorte qu'ils disposent dans l'état de montage d'un serrage par friction et/ou de forces d'action par engagement par adhérence ou par coopération de forme.
